(19) Europäisches Patentamt
European Patent Office
Office européen des brevets

(11) EP 0 900 191 B1

(12) **FASCICULE DE BREVET EUROPEEN**

(45) Date de publication et mention de la délivrance du brevet:
**04.09.2002 Bulletin 2002/36**

(21) Numéro de dépôt: **97921907.8**

(22) Date de dépôt: **30.04.1997**

(51) Int Cl.7: **C07C 209/84**, C07C 209/62, C01B 7/19

(86) Numéro de dépôt international:
**PCT/FR97/00766**

(87) Numéro de publication internationale:
**WO 97/041089 (06.11.1997 Gazette 1997/47)**

(54) **HYDROGENOFLUOROSULFONATES DE BASE ORGANIQUE, LEUR UTILISATION POUR LIBERER LES BASES ORGANIQUES DE LEUR FLUORHYDRATE, LEUR PROCEDE DE PREPARATION, COMPOSITION LES COMPORTANT**

HYDROGENFLUOROSULFONATE VON ORGANISCHEN BASEN, IHRE VERWENDUNG ZUR ENTFERNUNG VON FLUORWASSERSTOFF AUS ORGANISCHEN BASEN, VERFAHREN ZU IHRER HERSTELLUNG UND SIE ENTHALTENDE ZUSAMMENSETZUNGEN

ORGANIC BASE HYDROGENOFLUOROSULPHONATES, THEIR USE IN RELEASING ORGANIC BASES FROM THEIR FLUOROHYDRATE, METHOD OF PREPARATION THEREOF, COMPOUND CONTAINING THEM

(84) Etats contractants désignés:
**AT BE CH DE DK ES FI FR GB GR IE IT LI LU MC NL PT SE**

(30) Priorité: **30.04.1996 FR 9605573**

(43) Date de publication de la demande:
**10.03.1999 Bulletin 1999/10**

(73) Titulaire: **RHODIA CHIMIE**
**92408 Courbevoie Cédex (FR)**

(72) Inventeurs:
- **SAINT-JALMES, Laurent**
  **F-69330 Meyzieu (FR)**
- **MOREL, Marcel**
  **F-69530 Brignais (FR)**

(74) Mandataire: **Ricalens, François**
**Rhodia Services,**
**Direction de la Propriété Industrielle,**
**40, rue de la Haie-Coq**
**93306 Aubervilliers Cedex (FR)**

(56) Documents cités:
**EP-A- 0 121 466**
**DE-A- 2 719 577**
**GB-A- 2 138 810**
**US-A- 4 131 449**
**US-A- 4 481 370**

- **CHEMICAL ABSTRACTS, vol. 93, no. 17, 27 Octobre 1980 Columbus, Ohio, US; abstract no. 167055, ZIBAREV, A. V. ET AL: "Study of the behavior of pentafluorobenzoyliminosulfinyl dihalides in strong acids" XP002037177 & IZV. SIB. OTD. AKAD. NAUK SSSR, SER. KHIM. NAUK (1980), (2), 107-12,**
- **J. MED. CHEM. (1979), 22(3), 237-47, XP002037176 ROKACH, JOSHUA ET AL: "Cyclic amidine inhibitors of indolamine N-methyltransferase"**

Il est rappelé que: Dans un délai de neuf mois à compter de la date de publication de la mention de la délivrance du brevet européen, toute personne peut faire opposition au brevet européen délivré, auprès de l’Office européen des brevets. L’opposition doit être formée par écrit et motivée. Elle n’est réputée formée qu’après paiement de la taxe d’opposition. (Art. 99(1) Convention sur le brevet européen).



Printed by Jouve, 75001 PARIS (FR)

## Description

La présente invention a pour objet l'utilisation des fluorosulfonates pour libérer les bases organiques de leur fluorhydrate, des hydrogénofluorosulfonates de base organique, des procédés de préparation de fluorosulfonate(s), des compositions de fluorosulfonate(s) de base organique et d'acide fluorhydrique. Elle concerne plus particulièrement une technique permettant une séparation de l'acide fluorhydrique d'avec les bases organiques susceptibles de former des associations, constituant la plupart du temps des composés définis, avec plusieurs molécules d'acide fluorhydrique unitaire (c'est-à-dire HF), au moins trois.

Le procédé selon la présente invention est particulièrement bien adapté pour les bases constituant avec l'acide fluorhydrique, des associations comportant plus de deux ; avantageusement trois unités d'acide fluorhydrique.

Il est bien connu des spécialistes en la matière que les bases organiques forment, avec l'acide fluorhydrique, des complexes comportant au moins trois unités d'acide fluorhydrique. Généralement, plus les bases sont faibles et sont molles, plus le nombre d'unités d'acide fluorhydrique par fonction basique, augmente.

Or les composés fluorés en raison de leur propriétés spécifiques, sont de plus en plus demandés et mis en oeuvre dans des applications comme l'agriculture et la santé.

La synthèse des dérivés fluorés est souvent délicate et met en oeuvre souvent les échanges de divers substituants avec le fluor. Or le réactif le plus utilisé et le moins cher pour réaliser l'échange est indéniablement l'acide fluorhydrique.

En outre ce dernier acide est de plus en plus employé comme milieu réactionnel.

Certains précurseurs des bases organiques posent des problèmes de stabilité, ainsi les fluorures de carbamoyle présentent une instabilité thermique qui conduit à une formation de l'isocyanate ; ou à la formation de fluorophosgène (Hoechst, EP 639556), dont la manipulation est très délicate en raison de sa toxicité. Par ailleurs le fluorophosgène conduit à la perte de deux atomes de fluor.

C'est pourquoi un des buts de la présente invention est de fournir une forme des bases organique dont l'utilisation permette un récupération importante de l'acide fluorhydrique.

Un autre but de la présente invention est de fournir une forme du type précédent dont l'utilisation permette l'hydrolyse des fluorures de carbamoyle sans dégager les phosgènes et notamment des fluorophosgènes.

Un autre but de la présente invention est de fournir un procédé de préparation d'une forme du type précédent.

Un autre but de la présente invention est de fournir un procédé de préparation d'une forme du type précédent qui permette la libération de la base à partir de carbamoyle.

Un autre but de la présente invention est de fournir une composition comportant ladite base organique et au moins deux équivalents d'acide fluorhydrique par fonction basique, composition à partir de laquelle la récupération de l'acide fluorhydrique est aisée.

Ces buts et d'autres qui apparaîtront par la suite sont atteints au moyen d'une utilisation de la forme Hydrogénofluorosulfonate d'une base organique pour séparer d'avec elle l'acide fluorhydrique qui est associé à ladite base ou qui est associé à un de ses précurseurs.

Ainsi, au cours de l'étude qui a mené à la présente invention, il a été montré que l'acide fluorosulfonique ($HFSO_3$) était capable de déplacer les complexes entre les bases organiques et l'acide fluorhydrique permettant ainsi de récupérer l'acide fluorhydrique.

La présente invention présentent surtout un intérêt pour certaines bases notamment les bases phosphorées et surtout azotées.

Ainsi, lorsque ladite base est azotée, elle est avantageusement choisie parmi les amines (y compris cycliques), les imines (y compris les hétérocycles aromatiques).

Lorsque ladite base est phosphorée, elle est avantageusement choisie parmi les phosphines (y compris cycliques), et les hétérocycles aromatiques présentant un phosphore comme hétéroatome.

Plus la faculté de ladite base organique à s'associer à l'acide fluorhydrique est grande et plus le nombre d'unités d'acide fluorhydrique auxquels elle est susceptible de s'associer est élevé, plus l'intérêt de la présente invention croît.

Ainsi, il est avantageux que ladite base organique soit choisie parmi celles dont le pKa associé est au plus égal à 8, avantageusement à 7.

Ladite base est choisie parmi celles qui sont capables de former avec l'acide fluorhydrique des complexes dont le rapport entre l'acide fluorhydrique et la fonction basique (ou au moins l'une des fonctions basiques lorsque la molécule traitée comporte plusieurs fonctions basiques) est au moins égal à 5.

Ainsi que cela a été exposé plus haut, ladite base est associée à l'acide fluorhydrique sous la forme d'un de ses précurseurs.

Selon une mise en oeuvre particulièrement avantageuse de la présente invention, ledit précurseur est un précurseur qui libère ladite base en consommant une molécule d'eau. Cette caractéristique présente un avantage lors de la préparation in situ de l'anion fluorosulfonate, comme on le verra ultérieurement.

Selon une variante avantageuse de la présente invention, dans ledit précurseur la fonction qui se transformera en fonction basique est une fonction libérant un gaz comme par exemple de l'acide carbonique (ou bioxyde de carbone).

Lorsque la dite base est une amine (y compris une aniline) un bon exemple de fonction libérant un gaz (acide carbonique, ou bioxyde de carbone) est à trouver dans les dérivés de l'acide carbamique, tels que fonctions urée, carbamoyle [souvent sous la forme d'halogénure, le plus souvent fluorure] ou carbamate. Ces fonctions dérivent des fonctions isocyanates.

Compte tenu de son coût, de sa relative instabilité, et de son agressivité, il est avantageux que ledit hydrogénofluorosulfonate soit formé in situ.

C'est pourquoi un des buts de la présente invention est de fournir un procédé qui permette de libérer une base d'avec un de ses complexes avec l'acide fluorhydrique.

Ce but et d'autres qui apparaîtront par la suite sont atteints au moyen d'un procédé qui comporte une étape a) dans laquelle l'on forme le hydrogénofluorosulfonate de ladite base organique.

Avantageusement, ce procédé comporte après l'étape a), une étape b) dans laquelle on récupère l'acide fluorhydrique de la composition ainsi modifiée.

Il est possible de former l'hydrogénoflurosulfonate par une simple action de l'acide hydrogénoflurosulfonique sur le complexe entre l'acide fluorhydrique et ladite base organique. C'est certainement une voie satisfaisante lorsque l'on a sur le site une source peu chère d'un tel acide.

Toutefois il est avantageux et c'est un autre but de la présente invention, de pouvoir préparer du fluorosulfonate in situ, notamment à partir d'une composition contenant de l'acide fluorhydrique, éventuellement associé avec une base organique.

Ainsi, au cours de l'étude qui a mené à la présente invention, il a été montré que l'acide fluorosulfonique pouvait aisément être fabriqué in situ par action d'acide sulfurique ou de trioxyde de soufre (anhydride sulfurique ou SO3) sur des compositions organiques sensiblement anhydres contenant de l'acide fluorhydrique.

Lorsque ladite composition, est humide et/ou ne contient pas de déshydratant, alors il peut être conseillé d'utiliser anhydride sulfurique ou SO3 tel que, ou sous forme d'oléum.

Si ladite composition contient un déshydratant, alors il peut être avantageux d'utiliser de l'acide sulfurique, éventuellement contenant un peu d'eau (acide sulfurique (à une concentration au moins égale à 80 %, avantageusement à 90 %, souvent à 95 %). Ainsi dans cette mise en oeuvre de la présente invention, l'acide fluorosulfonique est fabriqué in situ par action d'acide sulfurique sur l'acide fluorhydrique de la composition en présence de déshydratant.

Avantageusement ledit déshydratant est un précurseur de la dite base.

Ladite base organique est une amine et ledit précurseur de la dite base est une fonction isocyanate ou une fonçtion qui en dérive (par exemple urée carbamoyle [Halogénure le plus souvent fluorure] ou carbamate).

La réaction de synthèse de l'anion fluorosulfonique est menée à une température comprise entre 0 °C et 100 °C température de distillation de l'HF, avantageusement comprise entre 0°C et 50°C, de préférence comprise entre 0°C et 20 °C.

Quoique l'on puisse imaginer de mettre en oeuvre ladite récupération de l'acide fluorhydrique de diverse manière, et notamment par extraction liquide, il est en général préféré que l'étape b) de récupération d'acide fluorhydrique, soit une distillation.

Le présent procédé est notamment intéressant pour le recyclage des réactifs acides base qui se développent de plus en plus, notamment les réactifs constitués d'hétérocycle(s) aromatique(s), et d'acide fluorhydrique, famille de réactifs dont le composé [pyridine, 10HF] peut être considéré comme le paradigme.

Il peut surtout être aussi notamment intéressant pour la libération de celles des dites bases organiques (notamment intéressant aussi les fluorosulfonates correspondants) qui comportent au moins un carbone ou un soufre fluorophore.

Avantageusement ladite base organique comporte comme carbone ou comme soufre fluorophore, respectivement au moins un carbone d'hybridation sp3 ou un SF5.

Avantageusement ladite base organique présente comme fonction basique au moins un atome d'azote ou de phosphore trivalent, atome, cela va sans dire, présentant un doublet disponible accepteur de proton.

Pour que le procédé soit notamment intéressant il faut que ladite base soit stable en milieu très acide et/ou très déshydratant (milieu proche des olea, [ou, comme certains le disent, des oléums]).

Un autre but de la présente invention est de fournir des composés qui permettent de libérer les bases organiques de leurs complexes avec plusieurs unités d'acide fluorhydrique et ce notamment à partir de compositions contenant une forte proportion d'acide fluorhydrique. En général, au moins deux unités d'acide fluorhydrique, avantageusement au moins 3, de préférence au moins 4 unités d'acide fluorhydrique.

Ces buts et d'autres qui apparaîtront par la suite sont atteints au moyen d'hydrogénofluorosulfonate de base organique ; il est souhaitable que ladite base organique dont l'acide associé présente au moins une fonction basique dont le pKa soit (mesuré ou ramené en phase aqueuse) au plus égal à 10, avantageusement à 8, de préférence à 6. Il est souhaitable que ce pKa soit positif avantageusement au moins égal à 1, de préférence au moins égal à 2. Ainsi,

on préfère les bases organiques dont le pKa est compris entre 1 et 8, de préférence entre 2 et 6 (bornes incluses).

Présentent un intérêt industriel particulier celles des bases organiques dont l'hydrogénofluorosulfonate est visé par la présente invention, qui sont porteuses d'au moins un atome de fluor, avantageusement d'au moins deux atomes de fluor. Ce ou au moins un de ces fluors est avantageusement porté par un atome ne présentant pas un caractère aromatique (c'est-à-dire par un atome ne constituant pas un chaînon d'un cycle aromatique) lequel atome fluorophore (c'est-à-dire porteur de fluor) lorsqu'il est carbone est de préférence d'hybridation $sp^3$; cet atome fluorophore outre l'atome de fluor qui lui donne son qualificatif porte avantageusement un ou deux (semblables ou différents) atomes d'halogène [de préférence choisis parmi les halogènes légers, c'est-à-dire chlore ou fluor].

En d'autres termes, ladite base organique dont l'hydrogénofluorosulfonate est visé par la présente invention, comporte avantageusement au moins un, de préférence au moins deux fluors. Il est également préférable qu'il y ait au moins un fluor sur un carbone d'hybridation $sp^3$, lequel porte avantageusement un ou deux (semblables ou différents) atomes d'halogène [de préférence choisis parmi les halogènes légers, c'est-à-dire chlore ou fluor]. Il est souhaitable ainsi que la base organique dont l'hydrogénofluorosulfonate est visé par la présente invention, présente comme atome fluorophore au moins un carbone ou au moins un chalcogène de rang atomique au moins égal à celui du soufre, sont plus particulièrement visées les bases organiques qui comportent comme atome(s) fluorophore(s) au moins un carbone d'hybridation sp3 ou un chalcogène (lequel bien entendu ne peut être oxygène) hexavalent, de préférence soufre (par exemple SF5).

Avantageusement ledit atome fluorophore (par exemple carbone d'hybridation sp3 ou un soufre hexavalent) porte au moins deux fluors.

Ladite base organique présente avantageusement comme atome porteur de la (ou d'une des) fonction basique (c'est-à-dire accepteuse de proton) un azote ou un phosphore trivalent.

Parmi les bases intéressantes on peut citer les hétérocycles aromatiques tels que les pyridines, y compris les quinoleines, éventuellement substituées y compris par des halogènes (chlore, fluor, brome,..) et porteuses indirectement, ou plus avantageusement directement, d'au moins atome fluorophore tel que défini ci-dessus.
avantageusement ces bases répondent à la formule suivante :

$$Ar\text{-}L\text{-}'A\text{-}F_q(R)_{(v\text{-}q)}$$

avec Ar représentant une base de nature aromatique dans lequel l'hétéroatome porteur de la (ou d'une des) fonction basique (c'est-à-dire accepteuse de proton) est ou bien intracyclique (comme c'est le cas de la pyridine ou de la quinoléine) ou bien exocyclique (comme dans les anilines) ;
avec L représentant un lien entre Ar et 'A ;
avec 'A représentant l'atome fluorophore tel que défini ci dessus ;
q représente le nombre de fluor portés par ledit atome fluorophore et est au moins égal à un et au plus égal à v ;
v représente la valence résiduelle (c'est-à-dire disponible après avoir pris en compte la liaison entre L et 'A) de l'atome fluorophore ;
les R, semblables ou différents, (bien sûr lorsque v>q) représentent des halogènes, des atomes d'hydrogène, une chaînes carbonée, peut comporter un ou plusieurs autres atomes fluorophores.
R peut s'accrocher à une position de Ar pour former un cycle (lequel cycle présente avantageusement de 4 à 8 chaînons, de préférence de 5 à 7 chaînons) ; dans ce cas R peut avantageusement prendre les valeurs de L et notamment être simplement un chalcogène (notamment oxygène et soufre) ou une simple liaison.
L est avantageusement choisi parmi une simple liaison, un atome de chalcogène, un radical carboné divalent pouvant comporter un ou plusieurs atomes fluorophores. Dans le cas d'un radical carboné divalent, la liaison entre L et Ar peut être une liaison carbone $sp^2$-chalcogène, par ailleurs il peut être avantageux que la liaison entre L et l'atome fluorophore soit une liaison chalcogène-atome fluorophore.

Ar peut être éventuellement substitué sur ses sommets disponibles, y compris par des halogènes (chlore, fluor, brome,..) et/ou par d'autres substituants répondant à la formule ; $-L-'A-F_q(R)_{(v-q)}$ explicitée ci dessus :
ainsi Ar représente une base de nature aromatique dans lequel l'hétéroatome porteur de la (ou d'une des) fonction basique (c'est-à-dire accepteuse de proton) est ou bien intracyclique (comme c'est le cas de la pyridine ou de la quinoléine) ou bien exocyclique (comme dans les anilines).

Ar présente avantageusement au plus environ 40 atomes de carbone, de préférence au plus environ 30, chaque R présente avantageusement au plus environ 10 atomes de carbone, de préférence au plus 8. L présente avantageusement au plus environ 10 atomes de carbone, de préférence au plus 8.

Le nombre total de carbone de la base est avantageusement d'au plus d'environ 50 atomes de carbone, de préférence d'au plus 30 atomes de carbone.

Il convient de signaler une sous famille de base celle où $'A-F_q(R)_{(v-q)}$ représente un radical perfluoré (c'est-à-dire $Rf = C_nF_{2n+1}$).

Il convient de signaler une sous famille de base celle où L représente une simple liaison ou un atome de chalcogène.

L'intersection entre ces deux sous familles est particulièrement intéressante.

La présente invention concerne aussi une composition comportant à la fois de l'acide fluorhydrique et un hydrogénosulfonate, composition où le rapport molaire entre l'acide fluorhydrique et les fonctions basiques de la dite base organique (HF/les fonctions basiques de la dite base organique) est au moins égal à 2, avantageusement à 3, de préférence à 4.

La teneur en eau de la dite composition est avantageusement telle que le nombre de molécule d'eau dans la composition soit au plus égal à la moitié (avantageusement aux quarts, de préférence au dixième) du nombre (exprimées en équivalent) de fonctions basiques présentes dans la composition.

En outre, la composition peut comporter aussi de l'acide sulfurique.

Bien sûr elle peut aussi comporter tous les produits utilisés dans les étapes précédentes de la synthèse de ladite base organique.

Les exemples non limitatifs suivants illustrent l'invention.

**<u>Généralités et mode opératoire généraux</u>**

L'analyse des bruts réactionnels a été réalisée par chromatographie phase gaz.

Principe

Comme cela est indiqué dans la description ci dessus, il est économiquement important et était techniquement envisageable de récupérer l'excès d'HF utilisé pour fluorer l'antéprécurseur de la pTFMA par distillation après l'étape de lyse du fluorure de carbamoyle en fluorhydrate de pTFMA:

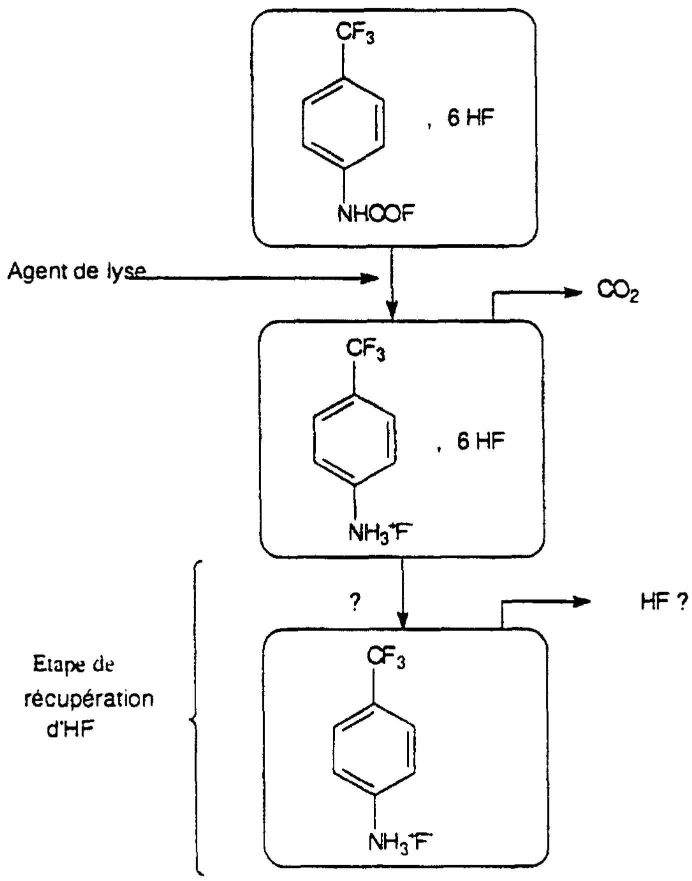


Après avoir en vain essayé de récupérer l'HF "libre" par distillation de mélange fluorhydrate de pTFMA / HF, on a testé la technique de lyse selon une mise en oeuvre particulièrement avantageuse de la présente invention, par

addition de l'acide sulfurique 98 % au mélange fluorhydrate de pTFMA / HF.

**Exemple 1 (comparatif) Distillation d'HF à partir d'un mélange fluorhydrate de pTFMA / HF**

Le dosage de cet échantillon (neutralisation, dosage pTFMA par HPLC, dosage des fluorures par chromatographie ionique) donne les proportions suivantes :

pTFMA / HF = 59/41

soit 5,6 équivalents d'HF par rapport à la pTFMA.

mode opératoire de la distillation

Expérimentalement le mélange fluorhydrate de pTFMA / HF a été chauffé dans un réacteur de 500 ml en téflon PFA sous courant d'azote. L'HF qui distille est piégé dans des barboteurs à potasse, les fluorures étant dosés par chromatographie ionique.

Le chauffage du "bouilleur" a été maintenu jusqu'à ce qu'un plafond de distillation de l'HF soit atteint sans dégradation de l'aniline trifluorométhylée

Les résultats sont rassemblés ci-dessous :

| N° essai | Fluorhydrate de pTFMA HF initial | T° | Durée | HF distillé | pTFMA* retrouvée |
|---|---|---|---|---|---|
| a | **pTFMA:** 0,198 mol<br>**HF**$_{libre}$ : 0,911mol | 60°C | 1H30 | 0,078 mole soit 8,5 % de l'HP "libre" initial | 96% |
| b | brut de a) | 90°C | 1H00 | 0,162 mole soit 19,5 % de l'HF "libre" restant | 100% |
| total | | | | 26 % de l'HF "libre" initial | 98 % |

*dosage par HPLC après neutralisation

Il apparaît que le chauffage du mélange fluorhydrate de pTFMA / HF ne permet de distiller que 26 % de l'HF "libre" (HF non sous forme de fluorhydrate) initialement présent.

L'HF restant lié à la pTFMA correspondrait à un complexe de formule approximative :

pTFMA, 4 HF

Ces complexes HF - Base sont connus pour être difficilement décomposés par simple chauffage.

En conclusion, le chauffage du brut réactionnel fluorhydrate de pTFMA / HF ne permet pas de recycler la totalité de l'HF présent initialement

**Exemple 2 Essais de récupération d'HF à partir du fluorure de carbamoyle, 6 HF**

Il a pu être observé que la simple addition d'acide sulfurique 98 % sur le fluorure de carbamoyle entraînait un dégagement gazeux important et immédiat .

L'analyse par infra-rouge du gaz formé démontre qu'il s'agit pour l'essentiel de l'anhydride carbonique ($CO_2$ ou bioxyde de carbone). il semblait que l'on se trouvât dans le cas précédent. Mais des analyses du brut réactionnel et des études ultérieures ont permis d'expliquer la formation de $CO_2$ par réaction entre le fluorure de carbamoyle et $H_2SO_4$ : l'acide sulfurique (même à 100 %) réagit avec pour former le fluorosulfonate de pTFMA et du $CO_2$.

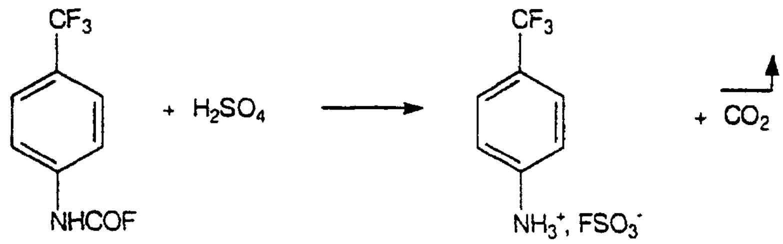


## Tableau II

### Traitement du mélange pTFMA, n HF par $H_2SO_4$

| Mélange de départ 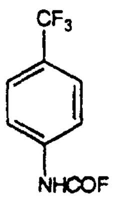 ; HF | $H_2SO_4$ : qualité et nb d'eqt par rapport à la ptfma | T°, durée | HF dégagé | RR pTFMA | HF non distillé |
|---|---|---|---|---|---|
| pTFMA;HF : 0,098 mol; HF : 0,629 mole soit 5,4 eqt, | $H_2SO_4$ 100 % 2,1 eqt | 60°C, 2 h. 90°C, 1 h. | 0,517 mole, soit 5,3 eqt | 98 % | 1,1 eqt d'HF % soit ≈ 0,1 eqt d'HF libre |
| pTFMA;HF : 0,097 mole; HF : 0,709 mole soit 6,3 eqt | $H_2SO_4$ 98 % 2,08 eqt % | 60 - 65°C, 2 h. 90°C, 1 h. | 0,65 mole soit 6,7 eqt | 97 % | HF: 1,16 eqt, soit 0,16 eqt HF libre |
| pTFMA;HF : 0,098 mole; HF 0,715 mole soit 6,3 eqt | $H_2SO_4$ 98 % 4 eqt | 65°C, 2 h. 90°C, 1 h. | 0,537 mole soit 5,48 eqt | 96,5 % | HF : 1,85 eqt, soit 0,85 d' HF libre |
| pTFMA;HF : 6,6 eqt HF | $H_2SO_4$ 98 % a) 1,13 eqt % 3 | 60 - 65°C 1H30 | | | HF : 1,47 eqt , soit 0,47 eqt HF libre |
| | b) 1 eqt supplé | 90°C 1H00 | | 98 % | HF:1 eqt, soit ≈ 0 eqt HF libre |

A la connaissance de la demanderesse, cette "lyse" sans eau d'une fonction halogénure de carbamoyle par de l'acide sulfurique n'a jamais été décrite dans la littérature.

Un mécanisme à 6 centres peut être proposé pour expliquer cette réaction, le groupement $CF_3$ activant fortement la fonction fluorure de carbamoyle:

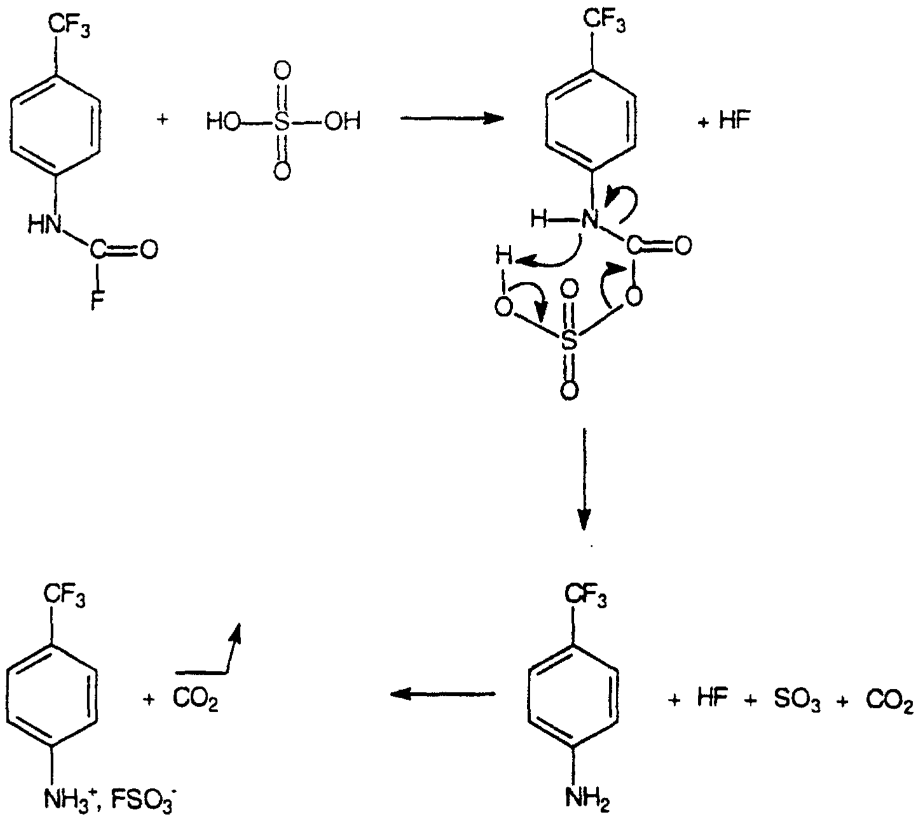

Le fluorosulfonate de pTFMA , caractérisé par RMN$^{19}$F et I.R. a été également synthétisé par action d'acide fluorosulfonique $HFSO_3$ sur la pTFMA

Le fluorosulfonate de pTFMA est un solide qui s'hydrolyse rapidement à l'humidité de l'air en hydrogénosulfate de pTFMA :

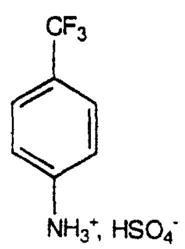

Expérimentalement, les essais résumés dans le tableau II ont été conduits de la manière suivante :

- De l'acide sulfurique 98 ou 100% a été additionné aux solutions précédentes à 20°C, entraînant un dégazage immédiat de $CO_2$.
- Après l'addition d'$H_2SO_4$, le mélange réactionnel homogène a été chauffé, sous courant d'azote, pour éliminer l'HF, celui-ci étant piégé dans des barboteurs à potasse. Les fluorures obtenus sont dosés par chromatographie ionique.
- Après retour à température ambiante les milieux réactionnels ont été analysés.

Après neutralisation anhydre à la pentylamine, la pTFMA a été dosée par HPLC.

Des résultats obtenus, on peut donc tirer les conclusions suivantes :

- l'action d'environ 2 équivalents d'$H_2SO_4$ (98 ou 100 %) sur un mélange , HF (5 à 6 équivalents d'HF par rapport au fluorure de carbamoyle), suivie d'un chauffage à 90°C permet d'éliminer la quasi totalité de l'HF "libre" initial.

- après élimination de l'HF le milieu obtenu est homogène liquide, et correspond à une solution de fluorosulfonate de pTFMA dans l'$H_2SO_4$ contenant un peu d'HF (0,1 à 0,2 équivalent de l'HF "libre" initial).

- l'utilisation de 4 équivalents d'$H_2SO_4$ 98 % limite le nombre d'équivalents d'HF distillé. Ceci peut s'expliquer par l'eau apportée par l'$H_2SO_4$ 98 %, qui peut hydrolyser le fluorure de carbamoyle en fluorhydrate de pTFMA . Dans ce cas des complexes HF - base peuvent se former entre et l'HF et limiter la distillation de l'HF, comme cela a déjà été remarqué.
  Ce type de complexe HF - base ne doit pas se former entre le fluorosulfonate de pTFMA et l'HF.

- après neutralisation du brut réactionnel dégazé d'HF, la pTFMA a été obtenue avec un rendement de l'ordre de 97 - 98 % (dosage HPLC).

En conclusion, l'addition de 2 équivalents d'$H_2SO_4$ 98 ou 100 % au brut de fluoration du pTCMI 2 permet par chauffage de recycler la quasi totalité de l'HF libre initialement présent et d'obtenir une solution homogène liquide de fluorosulfonate de pTFMA 7 dans environ 1 équivalent d'$H_2SO_4$.
A partir de la solution de fluorosulfonate de pTFMA dans l'acide sulfurique après distillation de l'HF, la pTFMA peut-être obtenue par neutralisation avec de la soude aqueuse et extraction au chlorure de méthylène. Le rendement de cette étape de neutralisation et d'extraction est supérieur à 98 %.

## Revendications

1. Utilisation de la forme Hydrogénofluorosulfonate d'une base organique pour séparer d'avec elle l'acide fluorhydrique qui est associé à ladite base ou qui est associé à un de ses précurseurs.

2. Utilisation selon la revendication 1, **caractérisée par le fait que** ladite base est azotée.

3. Utilisation selon les revendications 1 et 2, **caractérisée par le fait que** ladite base est choisie parmi les amines (y compris cycliques), les imines (y compris les hétérocycles aromatiques).

4. Utilisation selon les revendications 1 à 3, **caractérisée par le fait que** ladite base est choisie parmi les phosphines (y compris cycliques), et les hétérocycles aromatiques présentant un phosphore comme hétéroatomes.

5. Utilisation selon les revendications 1 à 4, **caractérisée par le fait que** ladite base est choisie parmi celles dont le pKa associé est au plus égal à 8, avantageusement à 7.

6. Utilisation selon les revendications 1 à 5, **caractérisée par le fait que** ladite base est choisie parmi celles qui sont capables de former avec l'acide fluorhydrique des complexes dont le rapport entre l'acide fluorhydrique et les fonctions basique est au mois égal à 5.

7. Utilisation selon les revendications 1 à 6, **caractérisée par le fait que** ladite base est associée à l'acide fluorhydrique sous la forme d'un précurseur.

8. Utilisation selon les revendications 1 à 7, **caractérisée par le fait que** ledit précurseur est un précurseur qui libère ladite base en consommant une molécule d'eau.

9. Utilisation selon les revendications 1 à 8, **caractérisée par le fait que** ledit précurseur est une fonction libérant de l'acide carbonique (ou bioxyde de carbone).

10. Utilisation selon les revenedications 1 à 9, **caractérisée par le fait que** ladite base est une amine (y compris une aniline) et ledit précurseur est une fonction libérant de l'acide carbonique (ou bioxyde de carbone).

11. Utilisation selon les revendications 1 à 10, **caractérisée par le fait que** ladite base est une amine et la fonction précurseur est une fonction isocyanate ou une fonction qui en découle (urée carbamoyle [Halogénure le plus souvent fluorure] ou carbamate).

**12.** Utilisation selon les revendications 1 à 11, **caractérisée par le fait que** ledit hydrogénofluorosulfonate est formé in situ.

**13.** Procédé de traitement d'une composition comportant une base organique ou son précurseur et de l'acide fluorhydrique anhydre, **caractérisé par le fait qu'**il comporte une étape a) dans laquelle l'on forme l'hydrogénofluorosulfonate de ladite base organique.

**14.** Procédé selon la revendication 13, **caractérisé par le fait qu'**il comporte après l'étape a), une étape b) dans laquelle on récupère l'acide fluorhydrique de la composition ainsi modifiée.

**15.** Procédé selon les revendications 13 et 14, **caractérisé par le fait que** l'acide fluorosulfonique est fabriqué in situ par action d'acide sulfurique ou de trioxyde de soufre ($SO_3$) sur ladite composition.

**16.** Procédé selon les revendications 14 et 15, **caractérisé par le fait que** l'acide fluorosulfonique est fabriqué in situ par action d'acide sulfurique sur l'acide fluorhydrique de la composition en présence de déshydratant.

**17.** Procédé selon la revendication 16, **caractérisé par le fait que** ledit déshydratant est le précurseur de la dite base.

**18.** Procédé selon la revendication 17, **caractérisé par le fait que** ladite base organique est une amine et ledit précurseur de ladite base est une fonction isocyanate ou une fonction qui en dérive (par exemple urée carbamoyle [Halogénure le plus souvent fluorure] ou carbamate).

**19.** Procédé selon la revendication 18, **caractérisé par le fait que** la réaction est menée à une température comprise entre 0°C et 100°C.

**20.** Procédé selon la revendication 19, **caractérisé par le fait que** ladite récupération de l'acide fluorhydrique de l'étape b) est une distillation.

**21.** Procédé selon les revendications 1 à 20, **caractérisé par le fait que** ladite récupération de l'acide fluorhydrique de l'étape b) est une extraction.

**22.** Composition comportant à la fois de l'acide fluorhydrique et un hydrogénofluorosulfonate d'une base organique comportant au moins un atome fluorophore choisi parmi les carbones d'hybridation $sp^3$ ou parmi les chalcogènes de rang et au moins égal à celui du soufre, ledit atome fluorophore portant au moins deux fluors et ladite base comportant au plus 50 atomes de carbone, **caractérisée par le fait que** le rapport molaire entre l'acide fluorhydrique et les fonctions basiques de ladite base organique, est au moins égal à 2, avantageusement à 3, de préférence à 4.

**23.** Composition selon la revendication 22, **caractérisée par le fait que** la teneur en eau de ladite composition est telle que le nombre de molécule d'eau dans la composition soit au plus égal à la moitié, avantageusement au quart, de préférence au dixième, du nombre de fonctions basiques présentes dans la composition.

**24.** Composition selon les revendications 22 et 23, **caractérisée par le fait que** la composition comporte aussi de l'acide sulfurique.

## Claims

**1.** Use of the hydrogen fluorosulphonate form of an organic base to separate from it the hydrofluoric acid which is combined with the said base or which is combined with one of its precursors.

**2.** Use according to claim 1, **characterized in that** the said base is a nitrogen base.

**3.** Use according to claims 1 and 2, **characterized in that** the said base is chosen from amines (including cyclic amines) and imines (including aromatic heterocycles).

**4.** Use according to claims 1 to 3, **characterized in that** the said base is chosen from phosphines (including cyclic phosphines) and aromatic heterocycles containing a phosphorus as hetero atoms.

**5.** Use according to claims 1 to 4, **characterized in that** the said base is chosen from those whose pKa in combined form is not more than 8, advantageously not more than 7.

**6.** Use according to claims 1 to 5, **characterized in that** the said base is chosen from those capable of forming complexes with hydrofluoric acid in which the ratio between the hydrofluoric acid and the basic functions is at least equal to 5.

**7.** Use according to claims 1 to 6, **characterized in that** the said base is combined with hydrofluoric acid in the form of a precursor.

**8.** Use according to claims 1 to 7, **characterized in that** the said precursor is a precursor which releases the said base by consuming a molecule of water.

**9.** Use according to claims 1 to 8, **characterized in that** the said precursor is a function which releases carbonic acid (or carbon dioxide).

**10.** Use according to claims 1 to 9, **characterized in that** the said base is an amine (including an aniline) and the said precursor is a function which releases carbonic acid (or carbon dioxide).

**11.** Use according to claims 1 to 10, **characterized in that** the said base is an amine and the precursor function is an isocyanate function or a function derived therefrom (carbamoyl urea [halide, usually fluoride] or carbamate).

**12.** Use according to claims 1 to 11, **characterized in that** the said hydrogen fluorosulphonate is formed in situ.

**13.** Process for treating a composition containing an organic base or its precursor and anhydrous hydrofluoric acid, **characterized in that** it includes a step a) in which the hydrogen fluorosulphonate of the said organic base is formed.

**14.** Process according to claim 13, **characterized in that** it includes, after step a), a step b) in which the hydrofluoric acid is recovered from the composition thus modified.

**15.** Process according to claims 13 and 14, **characterized in that** the fluorosulphonic acid is manufactured in situ by the action of sulphuric acid or sulphur trioxide ($SO_3$) on the said composition.

**16.** Process according to claims 14 and 15, **characterized in that** the fluorosulphonic acid is manufactured in situ by the action of sulphuric acid on the hydrofluoric acid of the composition in the presence of a dehydrating agent.

**17.** Process according to claim 16, **characterized in that** the said dehydrating agent is the precursor of the said base.

**18.** Process according to claim 17, **characterized in that** the said organic base is an amine and the said precursor of the said base is an isocyanate function or a function which is derived therefrom (for example carbamoyl urea [halide, usually fluoride] or carbamate).

**19.** Process according to claim 18, **characterized in that** the reaction is carried out at a temperature of between 0EC and 100EC.

**20.** Process according to claim 19, **characterized in that** the said recovery of the hydrofluoric acid in step b) is a distillation.

**21.** Process according to claims 1 to 20, **characterized in that** the said recovery of the hydrofluoric acid in step b) is an extraction.

**22.** Composition containing both hydrofluoric acid and a hydrogen fluorosulphonate of an organic base containing at least one fluorophoric atom chosen from $sp^3$-hybridized carbons or from chalcogens from a row at least equal to that of sulphur, the said fluorophoric atom carrying at least two fluorines and the said base containing not more than 50 carbon atoms, **characterized in that** the molar ratio between the hydrofluoric acid and the basic functions of the said organic base is at least equal to 2, advantageously equal to 3, preferably equal to 4.

23. Composition according to claim 22, **characterized in that** the water content of the said composition is such that the number of molecules of water in the composition is not more than half, advantageously than quarter, preferably than a tenth, of the number of basic functions present in the composition.

24. Composition according to claims 22 and 23, **characterized in that** the composition also contains sulphuric acid.

**Patentansprüche**

1. Verwendung der Hydrogenfluorosulfonatform einer organischen Base, um mit ihr Fluorwasserstoffsäure abzutrennen, die an die Base assoziiert ist oder die an eine ihrer Vorläufer assoziiert ist.

2. Verwendung nach Anspruch 1, **dadurch gekennzeichnet, daß** die Base stickstoffhaltig ist.

3. Verwendung nach den Ansprüchen 1 und 2, **dadurch gekennzeichnet, daß** die Base unter den Aminen (einschließlich der cyclischen), den Iminen (einschließlich der aromatischen Heterocyclen) ausgewählt wird.

4. Verwendung nach den Ansprüchen 1 bis 3, **dadurch gekennzeichnet, daß** die Base unter den Phosphinen (einschließlich der cyclischen) und den aromatischen Heterocyclen, die ein Phosphoratom als Heteroatom aufweisen, ausgewählt wird.

5. Verwendung nach den Ansprüchen 1 bis 4, **dadurch gekennzeichnet, daß** die Base unter denjenigen ausgewählt wird, deren assoziierter pKa höchstens 8, vorteilhaft höchstens 7, beträgt.

6. Verwendung nach den Ansprüchen 1 bis 5, **dadurch gekennzeichnet, daß** die Base ausgewählt wird unter denjenigen, die imstande sind, mit Fluorwasserstoffsäure Komplexe zu bilden, deren Verhältnis zwischen Fluorwasserstoffsäure und den basischen Funktionen wenigstens 5 beträgt.

7. Verwendung nach den Ansprüchen 1 bis 6, **dadurch gekennzeichnet, daß** die mit Fluorwasserstoffsäure assoziierte Base in Form eines Vorläufers vorliegt.

8. Verwendung nach den Ansprüchen 1 bis 7, **dadurch gekennzeichnet, daß** der Vorläufer ein Vorläufer ist, der die Base unter Verbrauch eines Moleküls Wasser freisetzt.

9. Verwendung nach den Ansprüchen 1 bis 8, **dadurch gekennzeichnet, daß** der Vorläufer eine Kohlensäure (oder Kohlendioxid) freisetzende Funktion ist.

10. Verwendung nach den Ansprüchen 1 bis 9, **dadurch gekennzeichnet, daß** die Base ein Amin (einschließlich ein Anilin) ist, und der Vorläufer eine Kohlensäure (oder Kohlendioxid) freisetzende Funktion ist.

11. Verwendung nach den Ansprüchen 1 bis 10, **dadurch gekennzeichnet, daß** die Base ein Amin ist und die Vorläuferfunktion eine Isocyanatfunktion oder eine sich hiervon ableitende Funktion (Carbamoylharnstoff [das häufigste Halogenid Fluorid] oder Carbamat) ist.

12. Verwendung nach den Ansprüchen 1 bis 11, **dadurch gekennzeichnet, daß** das Hydrogenfluorosulfonat in situ gebildet wird.

13. Verfahren zur Behandlung einer Zusammensetzung, die eine organische Base oder seinen Vorläufer und wasserfreie Fluorwasserstoffsäure umfaßt, **dadurch gekennzeichnet, daß** es eine Stufe a) beinhaltet, bei der man das Hydrogenfluorosulfonat besagter organischer Base bildet.

14. Verfahren nach Anspruch 13, **dadurch gekennzeichnet, daß** es nach der Stufe a) eine Stufe b) beinhaltet, bei der man die Fluorwasserstoffsäure der so modifizierten Zusammensetzung zurückgewinnt.

15. Verfahren nach den Ansprüchen 13 und 14, **dadurch gekennzeichnet, daß** die Fluorosulfonsäure in situ durch Einwirken von Schwefelsäure oder Schwefeltrioxid ($SO_3$) auf besagte Zusammensetzung gebildet wird.

16. Verfahren nach den Ansprüchen 14 und 15, **dadurch gekennzeichnet, daß** die Fluorosulfonsäure in situ durch

Einwirken von Schwefelsäure auf Fluorwasserstoffsäure der Zusammensetzung in Anwesenheit eines Dehydratationsmittels gebildet wird.

**17.** Verfahren nach Anspruch 16, **dadurch gekennzeichnet, daß** das Dehydratationsmittel der Vorläufer der besagten Base ist.

**18.** Verfahren nach Anspruch 17, **dadurch gekennzeichnet, daß** die organische Base ein Amin ist und der Vorläufer der Base eine Isocyanatfunktion oder eine sich hiervon ableitende Funktion (z.B. Carbamoylharnstoff [das häufigste Halogenid Fluorid] oder Carbamat) ist.

**19.** Verfahren nach Anspruch 18, **dadurch gekennzeichnet, daß** die Reaktion bei einer Temperatur zwischen 0°C und 100°C durchgeführt wird.

**20.** Verfahren nach Anspruch 19, **dadurch gekennzeichnet, daß** die Rückgewinnung der Fluorwasserstoffsäure von Stufe b) eine Destillation ist.

**21.** Verfahren nach den Ansprüchen 1 bis 20, **dadurch gekennzeichnet, daß** die Rückgewinnung der Fluorwasserstoffsäure von Stufe b) eine Extraktion ist.

**22.** Zusammensetzung, umfassend gleichzeitig Fluorwasserstoffsäure und ein Hydrogenfluorosulfonat einer organischen Base, die zumindest ein Fluor-tragendes Atom, ausgewählt unter den Kohlenstoffen mit $sp^3$-Hybridisierung oder unter den Chalkogenen von der Rangordnung und zumindest gleich derjenigen des Schwefels, wobei das Fluortragende Atom zumindest zwei Fluor trägt und die Base höchstens 50 Kohlenstoffatome aufweist, **dadurch gekennzeichnet, daß** das Molverhältnis zwischen der Fluorwasserstoffsäure und den basischen Funktionen der organischen Base zumindest gleich 2, vorteilhaft zumindest gleich 3, vorzugsweise zumindest gleich 4, ist.

**23.** Zusammensetzung nach Anspruch 22, **dadurch gekennzeichnet, daß** der Wassergehalt der Zusammensetzung derart ist, daß die Anzahl der Wassermoleküle in der Zusammensetzung höchstens gleich der Hälfte, vorteilhaft dem Viertel, bevorzugt dem Zehntel, der Anzahl der in der Zusammensetzung vorhandenen basischen Funktionen ist.

**24.** Zusammensetzung nach den Ansprüchen 22 und 23, **dadurch gekennzeichnet, daß** die Zusammensetzung auch Schwefelsäure enthält.